# EUROPEAN PATENT APPLICATION

(11) **EP 4 714 368 A1**
(43) Date of publication of application: **25.03.2026**
(21) Application number: 24810313.7
(22) Date of filing: 17.05.2024
(51) Int. Cl.: A61B 17/072, H02J 7/00, H01H 1/06

(54) **SURGICAL INSTRUMENT AND ASSEMBLING METHOD THEREFOR**

(30) Priority: 19.05.2023 CN 202310566680
(71) Applicant: Ezisurg Medical Co., Ltd., Shanghai 201315 (CN)
(72) Inventor: TANG, Chuangang, Shanghai 201315 (CN); YANG, Guang, Shanghai 201315 (CN); NIE, Honglin, Shanghai 201315 (CN)
(74) Representative: Daub, Thomas
(86) International application number: PCT/CN2024/093847
(87) International publication number: WO 2024/240067

(57) **Abstract**

The present application relates to a surgical instrument and an assembling method therefor. A power source discharging mechanism includes a circuit board and a sliding member. The circuit board includes a discharging circuit, a conductive sheet, and a conductive contact surface. A fixed terminal of the conductive sheet is fixed to the circuit board, and the conductive contact surface faces an extension section of the conductive sheet. The sliding member is opposite to the conductive sheet and can linearly move relative to the power source assembly. At a first position, at least a portion of the extension section of the conductive sheet is separated from the conductive contact surface. At a second position, at least a portion of the extension section of the conductive sheet is in contact with the conductive contact surface. The discharging circuit in the present application is turned on or turned off only based on a contact/separation relationship between the conductive sheet and the conductive contact surface, which is no longer limited to contact/separation between paired contact terminals of a discharging mechanism and a power source.

## Description

### CROSS-REFERENCE TO RELATED APPLICATION

This application claims priority to Chinese Patent Application No. 202310566680.2, filed with the China National Intellectual Property Administration on May 19, 2023 and entitled "SURGICAL INSTRUMENT AND ASSEMBLING METHOD THEREFOR", which is incorporated herein by reference in its entirety.

### FIELD

The present application relates to the field of medical instruments, and more particularly, to a power source discharging mechanism for a surgical instrument, and a corresponding surgical instrument and an assembling method therefor.

### BACKGROUND

At present, in various medical application scenarios, it can be seen that various types of surgical instruments are put into use, particularly surgical instruments with detachable power source assemblies.

To ensure that a power source of the surgical instrument contains electric energy required in a treatment process when the surgical instrument is used to treat a single patient, each detachable power source assembly is typically used only in a single treatment process. After use of the surgical instrument, a power source component usually needs to be separated from a main device, and the power source component is fully discharged within next 24 hours. This can ensure that there is no cross-contamination caused by mixing of the power source component and other main devices, and can also effectively recycle and dispose a fully discharged power source component. If the power source component is discarded directly without being separated from the main device and a battery is not discharged promptly, it may cause power liquid leakage or explosion. To avoid the aforementioned safety hazard, it is necessary to arrange a discharging mechanism inside the surgical instrument. The discharging mechanism is typically configured as follows: in one aspect, the discharging efficiency of the discharging mechanism is that electric energy of a power source is used up within several hours since when the power source is assembled to the corresponding surgical instrument; and in another aspect, even if the discharging mechanism runs simultaneously with the main device of the surgical instrument, the discharging mechanism will not affect the running performance of the main device.

The discharging mechanism of a medical device in the existing art is usually configured independently of a contact terminal of the main device of the surgical instrument, which means that contact terminals are additionally provided for these discharging mechanisms, such as elastic contact sheets or claws. Further, based on a fundamental principle of a circuit design, the additionally provided contact terminals imply that the required contact terminals are necessarily paired, rather than individual units. For example, the patent CN103096818B of Ethicon, Inc. features a discharging drain electrode that can translate. When a battery pack is attached to a battery base, a protruding member on the battery base is in contact with the discharging drain electrode, and the discharging drain electrode translates relative to a housing to electrically connect a first positive electrode of the battery pack to a first negative electrode of the battery. In this patent, the discharging drain electrode needs to have a first group of contacts and a second group of contacts that are electrically connected to a first group of batteries and a second group of batteries, respectively. If a group of contacts are not electrically connected to the battery pack, the battery pack will not be discharged successfully. Furthermore, the movable discharging drain electrode can easily be damaged in a process of plugging and unplugging the battery pack, leading to poor contact. The patent CN212991187U of Shandong Weirui Surgical Medical Products Co., Ltd. discloses an electric anastomat and a self-discharge battery pack. A first/second elastic plate of a discharging mechanism and a corresponding short-circuit contact sheet, as well as a corresponding input terminal contact sheet, are arranged in pairs. The discharging mechanism can also move a preset distance in an axial direction of the mounting hole. The patent CN112236090A of Tianjin Rich Surgical Instruments Co., Ltd. discloses a surgical instrument and a battery pack thereof. When paired switching components are operated to switch to a closed state, a discharging element is electrically connected to a battery element of the battery pack, and the switching components have an initial open state in which the discharging circuit is not turned on, an intermediate open state in which the switching components cooperate with the protruding portion to cause the discharging circuit to not be turned on, and a closed state in which the switching components are separated from the protruding portion to cause the discharging circuit to be turned on. The discharging circuit can complete discharging only when a battery is separated from a main device. The patent CN216085162U of Ningbo Haitai Kemai Medical Devices Co., Ltd. discloses a surgical instrument and a battery assembly thereof. The battery assembly is in contact with a corresponding positive electrode connection sheet and negative electrode connection sheet of an electric anastomat through a pair of sheet portions that can elastically deform. A discharging plate assembly is configured as follows: when the switch element is in an open state, the discharging circuit is not electrically connected to the battery element, so that the discharging circuit is not turned on; when the switch element is in a closed state, the discharging circuit is electrically connected to the battery element, so that the discharging circuit is turned on to discharge the battery element; and a switch element switching mechanism is configured to be operated to cause the switch element to switch from the open state to the closed state. This structure is relatively complex and requires the switch switching mechanism.

Therefore, the inventor of the present application expects to develop a new power source discharging mechanism for a surgical instrument. This mechanism can implement electrical connection to a power source without additionally providing paired contact terminals, and can also ensure that discharging can be completed within 24 hours without removing the battery from the main device, thus ensuring the safety of the battery. Furthermore, the structure is simple, and it is not likely to cause a contact failure. A new idea is provided for the design of the discharging mechanism.

### SUMMARY

To solve the above technical problems, in a first aspect, the present application provides a surgical instrument, including a power source assembly, a power source assembly base for accommodating the power source assembly, and a power source discharging mechanism. The power source discharging mechanism is mounted on the power source assembly. The power source discharging mechanism includes:
a circuit board, which includes a discharging circuit, a conductive sheet, and a conductive contact surface that are integrated onto the circuit board, where the conductive sheet includes a fixed terminal and an extension section; the fixed terminal of the conductive sheet is electrically connected to a first terminal of the discharging circuit; the extension section of the conductive sheet and the conductive contact surface switch between contact conduction and disconnection; the conductive contact surface is electrically connected to a second terminal of the discharging circuit; and
a sliding member, which is able to slide between a first position and a second position, where when the sliding member is at the first position, the extension section of the conductive sheet is disconnected from the conductive contact surface, and the discharging circuit is in a turned-off state; and when the sliding member is at the second position, the extension section of the conductive sheet is in contact conduction with the conductive contact surface, and the discharging circuit is in a turned-on state.

In an embodiment, the fixed terminal is constructed as being rigid, and at least a portion of the extension section is constructed as being flexible, to cause the extension section to deflect relative to the fixed terminal and make the conductive sheet to be in contact with the conductive contact surface.

In another embodiment, the extension section includes a transition section and an end section; the transition section extends in a direction parallel to a linear motion direction of the sliding member, and connects the fixed terminal to the end section; and the transition section is farther away from the sliding member in a vertical direction than the end section.

In still another embodiment, the sliding member has a first working surface and a second working surface; when the sliding member is at the first position, a tip end of the end section is located on the first working surface; when the sliding member is at the second position, the tip end of the end section is located on the second working surface; and a height of the first working surface is lower than a height of the second working surface in the vertical direction.

In still another embodiment, the extension section is designed to cause the extension section and the conductive contact surface to be in a form of a normally closed switch.

In still another embodiment, the sliding member includes a first protrusion designed to be located between the extension section and the conductive contact surface when the sliding member is at the first position, to ensure disconnection between the extension section and the conductive contact surface.

In some embodiments, a second protrusion facing the conductive sheet is arranged on the second working surface of the sliding member; and when the sliding member is at the second position, the second protrusion prevents the sliding member from sliding towards the first position.

In still another embodiment, the conductive contact surface is positioned at a location on the circuit board where, when the sliding member is at the second position, at least a portion of the extension section of the conductive sheet presses against the circuit board.

In still another embodiment, the circuit board further includes an input terminal and an output terminal that are respectively configured to be electrically connected to a positive electrode of a power source and a negative electrode of the power source; the input terminal is electrically connected to a third terminal of the discharging circuit; the output terminal is electrically connected to a fourth terminal of the discharging circuit; and the discharging circuit includes a discharging resistor.

In still another embodiment, the power source assembly base includes a first electrical contact terminal and a second electrical contact terminal; the first electrical contact terminal is configured to be electrically connected to the input terminal of the circuit board; and the second electrical contact terminal is configured to be electrically connected to the output terminal of the circuit board.

In some embodiments, the power source assembly base further includes a abutting top portion for pressing against the sliding member, so that when the power source assembly is assembled to the surgical instrument, the sliding member slides relative to the power source assembly, and the abutting top portion has the same shape as or a shape similar to a cross-sectional shape of the sliding member.

In a second aspect, the present application provides an assembling method for the surgical instrument according to the first aspect of the present application, where the power source assembly base has a guide rail, and the assembling method includes:
- placing the power source assembly at a first position of the power source assembly base, wherein the abutting top portion is separated from the sliding member, a tip end of the end section of the conductive sheet is put on the first working surface of the sliding member, and the extension section of the conductive sheet is separated from the conductive contact surface, to cause the discharging circuit to be in a turned-off state;
- pushing the power source assembly forwards along the guide rail to reach a middle position, where the abutting top portion presses against the sliding member and remains relatively stationary with the sliding member; the second protrusion of the sliding member presses against the end section of the extension section and forces the conductive sheet to elastically deform; and the extension section deflects relative to the fixed terminal until the extension section presses against the conductive contact surface, to cause the discharging circuit to be in a turned-on state; and
- continuing to push the power source assembly forwards along the guide rail to reach a second position, where the second protrusion moves and crosses the tip end of the end section to prevent the sliding member from moving towards the first position and then prevent the conductive sheet from being disconnected from the conductive contact surface, to cause the discharging circuit to remain in the turned-on state.

Compared with the existing art, the power source discharging mechanism and the corresponding surgical instrument that are provided in the present application have the following advantages: first, the discharging circuit aimed at discharging a power source is designed to be turned on or turned off only based on a contact/separation relationship between a switch composed of the conductive sheet, and the conductive contact surface, which is no longer limited to contact/separation between paired contact terminals of a discharging mechanism and the power source. Second, considering structural configuration of the circuit board in the present application, it is not necessary to additionally provide contact terminals that are independent of a main device of the surgical device. Furthermore, either the main device of the surgical device or the discharging circuit can obtain electric energy from the power source by virtue of the input terminal and the output terminal of the circuit board. Further, the discharging circuit is integrated onto the circuit board, which eliminates additional arrangement of components for discharging. In the design aspect, a space and used components and materials are significantly saved. Still further, after a battery is mounted, discharging can be completed within 24 hours even if the battery is not removed from the main device. This ensures the safety of the battery.

### BRIEF DESCRIPTION OF THE DRAWINGS

For clearer descriptions of the technical solutions according to the embodiments of the present application, the drawings required to be used in the description of the embodiments are briefly introduced below. It is obvious that the drawings in the description below are only some embodiments of the present application, and it is obvious for those skilled in the art that other drawings can be acquired according to the drawings without creative efforts.
FIG. 1 is a front view of a surgical instrument according to an embodiment of the present application;
FIG. 2 is an exploded view of a power source assembly and a power source assembly base of the surgical instrument according to the embodiment shown in FIG. 1;
FIG. 3 is a front view of a circuit board according to an embodiment of the present application;
FIG. 4 is a planar graph of a surface of a circuit board that faces a switch according to an embodiment of the present application;
FIG. 5 is a front view of a circuit board and a sliding member according to an embodiment of the present application;

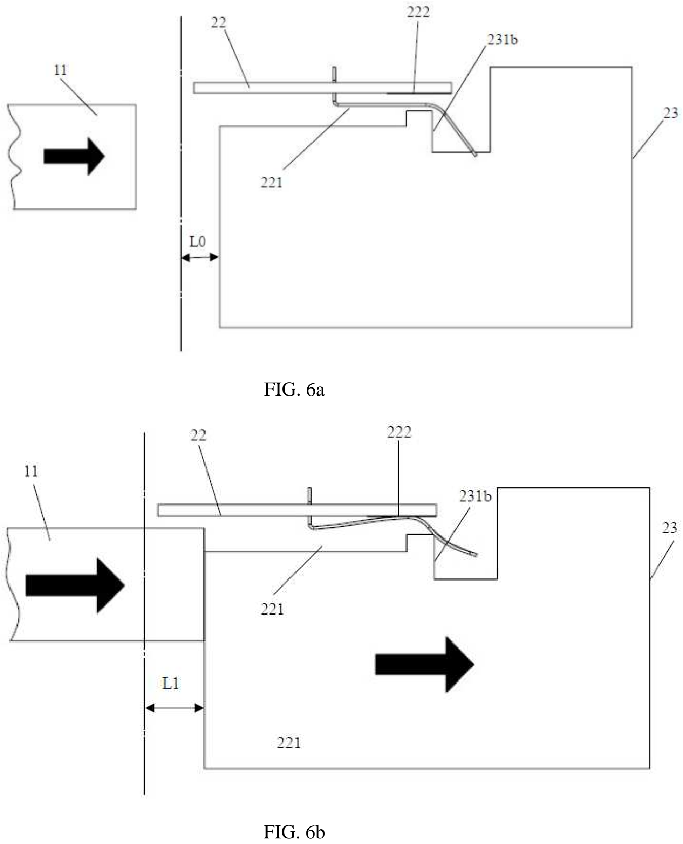

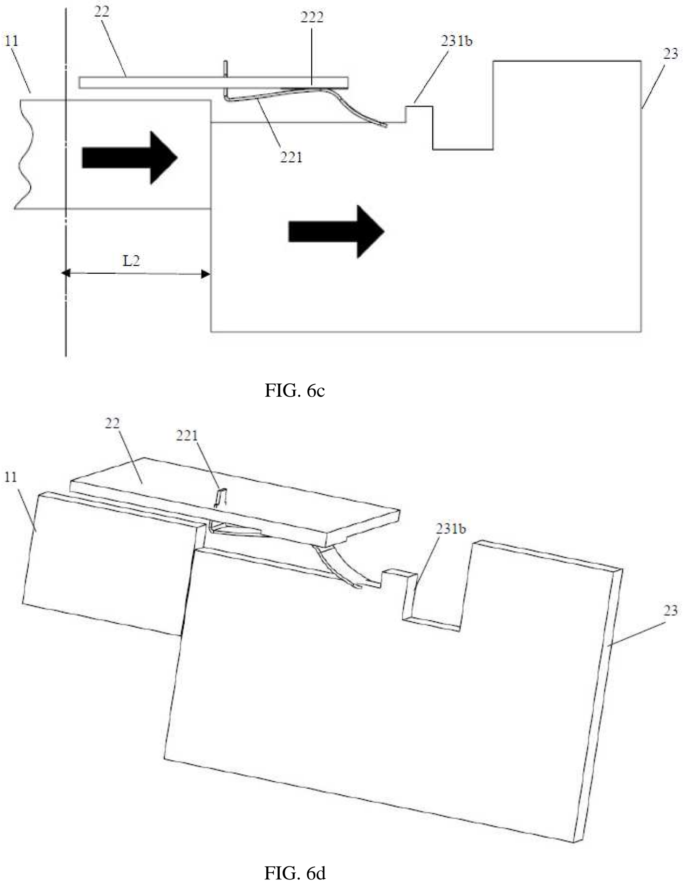

FIG. 6a, FIG. 6b, and FIG. 6c are respectively front views of a sliding member that is at a first position, a middle position, and a second position according to an embodiment of the present application, where L0 represents an initial distance between the sliding member that is at the first position and a reference line; L1 represents a distance between the sliding member that is at the middle position and the reference line; L2 represents a distance between the sliding member that is at the second position and the reference line; FIG. 6d is a perspective view of the sliding member that is at the second position;
FIG. 7 is a perspective view of cooperation between a abutting top portion of a power source assembly base and a sliding member of a power source assembly according to an embodiment of the present application, where other components are omitted; and
FIG. 8a, FIG. 8b, and FIG. 8c are respectively front views of a sliding member that is at a first position, a middle position, and a second position according to another embodiment of the present application, where L0' represents an initial distance between the sliding member that is at the first position and a reference line; L1' represents a distance between the sliding member that is at the middle position and the reference line; and L2' represents a distance between the sliding member that is at the second position and the reference line.

List of reference numerals in the accompanying drawings:
1: surgical instrument; 11: abutting top portion; 12: power source assembly base; 2: power source assembly; 21: front housing; 22: circuit board; 221: conductive sheet; 221a: fixed terminal; 221b, 221b': extension section; 221b-1, 221b-1': transition section; 221b-2, 221b-2': end section; 221b-3, 221b-3': tip end of end section; 222: conductive contact surface; 223: input terminal; 224: output terminal; 225: main device power supply terminal; 23, 23': sliding member; 231a: first protrusion; 231b: second protrusion; 232: first working surface of sliding member; 233: second working surface of sliding member; 24: power source; 25: rear housing; L0, L0', L1, L1', L2, L2': distance.

### DETAILED DESCRIPTION

**In** order to make the objectives, technical solutions, and advantages of the present application clearer, the technical solutions in the embodiments of the present application are clearly and completely described below with reference to the accompanying drawings in the embodiments of the present application. Apparently, the described embodiments are merely some rather than all of the embodiments of the present application. All other embodiments obtained by a person of ordinary skill in the art based on the embodiments of the present application without making creative efforts shall fall within the protection scope of the present application.

In the descriptions of the present application, it should be understood that orientations or positional relationships indicated by the terms "X axis", "Y axis", "Z axis", "vertical", "parallel", "upper", "lower", "front", "rear", "left", "right", "vertical", "horizontal", "top", "bottom", "inner", "outer", and the like are orientations or positional relationships as shown in the drawings, and are only for the purpose of facilitating and simplifying the descriptions of the present application instead of indicating or implying that devices or elements indicated must have particular orientations, and be constructed and operated in the particular orientations, so that these terms are not construed as limiting the present application. In addition, the terms "first" and "second" are used for descriptive purposes only and are not to be construed as indicating or implying relative importance or to implicitly indicate the number of technical features indicated. From this, features defined as "first" and second" may explicitly or implicitly include one or more features. In the descriptions of the present application, "a plurality" means two or more, unless otherwise specified.

In the description of the present application, it should be noted that unless otherwise specified and limited, the terms "mount", "connect", and "connection" should be broadly understood. For example, it can be a fixed connection, detachable connection, integrated connection, mechanical connection, electrical connection, direct connection, indirect connection via an intermediate element, or internal communication between two elements. A person of ordinary skill in the art can understand the specific meanings of the above terms in the present application according to specific situations.

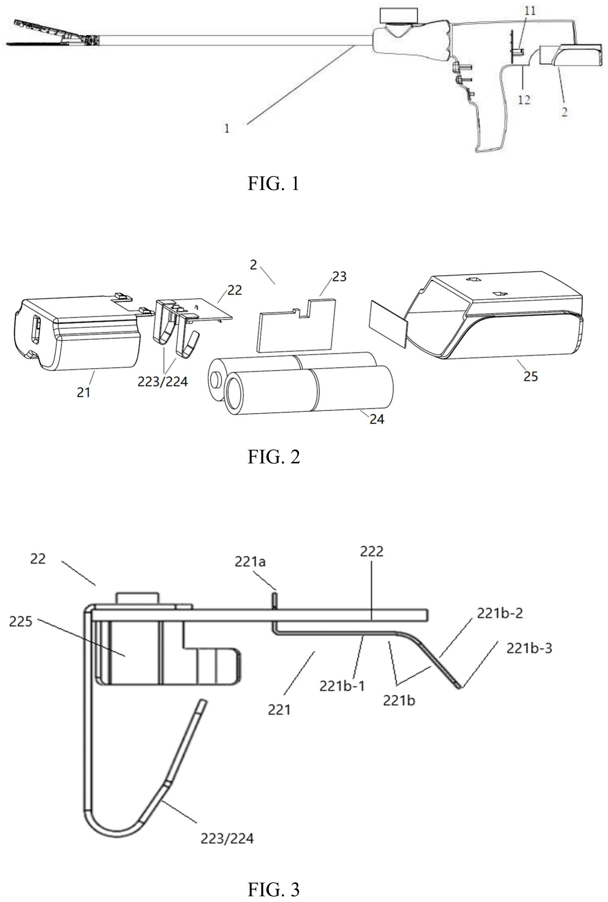

Referring to FIG. 1, a surgical instrument 1 according to an embodiment of the present application is shown. A power source assembly 2 is mounted to the surgical instrument 1 through a power source assembly base 12. For example, the power source assembly 2 slides into the power source assembly base 12 from back to front with the help of a guide rail in the power source assembly base, thereby assembling the power source assembly 2.

Referring to FIG. 2, an exploded view of a power source assembly 2 according to an embodiment of the present application is shown. A front housing 21 faces one side of the surgical instrument 1, and a circuit board 22 is assembled to an inner side of the front housing 21. The circuit board 22 is provided with contact terminals for taking electricity for the surgical instrument 1 and a discharging circuit, so the circuit board 22 is closely adjacent to a power source 24 in space. In this embodiment, the power source 24 employs a plurality of cells which are symmetrically distributed in two columns inside the power source assembly 2. Therefore, a sliding member 23 can be arranged between the power sources 24 and can linearly move along a slidable assembling direction of the sliding member 23 relative to the power source assembly 2. A rear housing 25 is provided with an electrode connector for connecting a corresponding electrode at one end of the power source 24 that is close to the rear housing 25. Assembling is performed based on the above interrelationships to obtain the power source assembly 2.

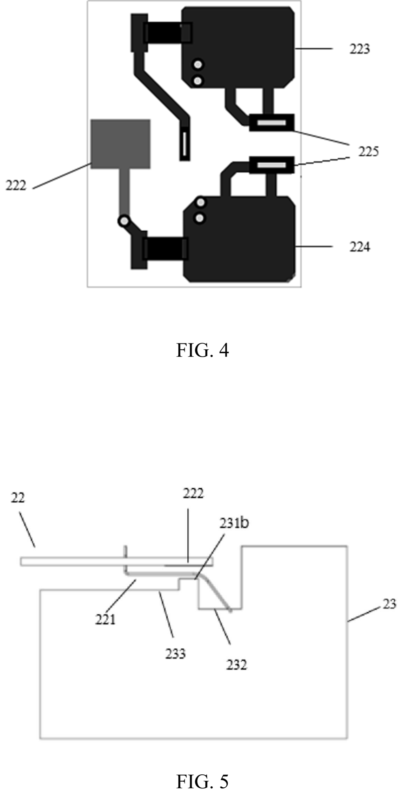

The structure of the circuit board 22 will now be described with reference to FIG. 3 and FIG. 4. The circuit board 22 includes a discharging circuit, a conductive sheet 221, and a conductive contact surface 222 that are integrated onto the circuit board 22. It should be noted that the discharging circuit integrated onto the circuit board 22 is not an entirety of the discharging circuit. Therefore, the discharging circuit on the circuit board 22 partially essentially has a plurality of terminals for being connected to other electronic elements beside the circuit board 22, including but not limited to: a first terminal, a second terminal, a third terminal, and a fourth terminal, which will be described below. The first terminal is electrically communicated to one of the third terminal and the fourth terminal (such as the third terminal), while the second terminal is electrically communicated to the other one of the third terminal and the fourth terminal (such as the fourth terminal). Thus, the first terminal and the third terminal, as well as a circuit wiring between them, form a part of path of the discharging circuit, while the second terminal and the fourth terminal, as well as a circuit wiring between them, form another part of path of the discharging circuit. In addition, the conductive sheet 221 includes a fixed terminal 221a and an extension section 221b. The fixed terminal 221a is fixed to the circuit board 22 in a manner of being intersected with (e.g. orthogonal to) the circuit board 22 and is electrically connected to the first terminal of the discharging circuit. The conductive contact surface 222 is electrically connected to the second terminal of the discharging circuit. The fixed terminal 221a is constructed as being rigid, and at least a portion of the extension section 221b is constructed as being flexible, to cause the extension section 221b to deflect relative to the fixed terminal 221a. More specifically, the extension section 221b includes a transition section 221b-1, an end section 221b-2, and a tip end 221b-3 of end section. The transition section 221b-1 extends in a direction parallel to a linear motion direction of the sliding member and connects the fixed terminal 221a to the end section 221b-2. The transition section 221b-1 is farther away from the sliding member 23 in a vertical direction than the end section 221b-2. The end section 221b-2 is designed to extend towards the rear housing 25 in a non-horizontal manner, but it gradually approaches the sliding member 23 in the vertical direction while extending towards the rear housing 25, thus presenting inclined downward extension shown. The transition section 221b-1 is constructed as being relatively flexible, and the end section 221b-2 is constructed as being relatively rigid. The tip end 221b-3 of end section is not fixedly connected to the circuit board 22. In addition, the fixed terminal 221a and the extension section 221b are conductive relative to each other, as further explained below.

The circuit board 22 includes an input terminal 223 and an output terminal 224 that are respectively configured to be electrically connected to a positive electrode of the power source 24 and a negative electrode of the power source 24. The input terminal 223 is electrically connected to the third terminal of the discharging circuit, and the output terminal 224 is electrically connected to the fourth terminal of the discharging circuit. The discharging circuit includes a discharging resistor. Moreover, the power source assembly base 12 includes a first electrical contact terminal and a second electrical contact terminal (not shown) that extend from the power source assembly base 12 towards the power source assembly 2 to take electricity. Each of the input terminal 223 and the output terminal 224 further includes a main device power supply terminal 225 (such as a protruding tongue), so that the first electrical contact terminal and second electrical contact terminal of the power source assembly base 12 can be electrically connected to the corresponding main device power supply terminals 225 respectively, for example, clamping the protruding tongues. Therefore, in one aspect, the discharging circuit obtains electric energy from the power source 24 by respectively communicating the third terminal and the fourth terminal to the input terminal 223 and the output terminal 224, to achieve a purpose of using up the electric energy of the power source 24. In another aspect, the first electrical contact terminal and second electrical contact terminal of the power source assembly base 12 are in contact conduction with the paired main device power supply terminals 225 to take electricity from the power source, thus achieving an expected medical treatment purpose.

Referring to FIG. 5, the sliding member 23 can be set to be in an irregular plate shape or can be in other suitable shapes in this embodiment. The sliding member 23 is spatially opposite to the conductive sheet 221 and can perform linear motion as described above. Specifically, the sliding member 23 has a first working surface 232 and a second working surface 233. The first working surface 232 is located at a portion close to the rear housing 25, and the second working surface 233 is located at a portion close to the front housing 21. A height of the first working surface 232 is lower than a height of the second working surface 233 in a vertical direction. Still further, a second protrusion 231b facing the conductive sheet 221 is arranged on the second working surface 233 of the sliding member 23.

The conductive contact surface 222 is arranged on a surface of the circuit board 22 that faces the extension section 221b of the conductive sheet 221, and its position is as follows: when the sliding member 23 is at the second position, at least a portion of the extension section 221b of the conductive sheet 221 presses against the circuit board 22, which will be described in detail below. Therefore, the position of the conductive contact surface 222 depends on a shape of the conductive sheet 221 and cooperation between the conductive sheet 221 and the circuit board 22.

Referring specifically to FIG. 6a, it can be seen that when the sliding member 23 is at the first position, that is, when the sliding member 23 is at its starting position, the second protrusion 231b corresponds to the transition section 221b-1 of the conductive sheet 221 in the vertical direction. In this case, since the transition section 221b-1 is far away from the sliding member 23 in the vertical direction, the second protrusion 231b does not press against the transition section 221b-1 of the conductive sheet 221. The conductive sheet 221 is in its original state, and a tip end 221b-3 of end section of the conductive sheet 221 is put on the first working surface 232. The extension section 221b of the conductive sheet 221 is separated from the conductive contact surface 222, and the discharging circuit is thereby turned off.

Referring to FIG. 6b, in an assembling process that the power source assembly 2 slides into the power source assembly base along the above guide rail, the power source assembly 2 is at the middle position. The power source assembly 2 linearly moves forwards relative to the power source assembly base, and the abutting top portion 11 of the power source assembly base presses against the sliding member 23, to cause the sliding member 23 to remain stationary relative to the surgical instrument 1. In other words, in the process that the sliding member 23 linearly moves towards its second position, the sliding member 23 moves linearly relative to the power source assembly 2 towards the rear housing 25. In this case, the second protrusion 231b presses against the end section 221b-2 of the extension section 221b of the conductive sheet 221 and forces the conductive sheet 221 to elastically deform. In this case, since the end section 221b-2 gradually approaches the sliding member 23 and the second protrusion 231b of the sliding member 23 in the vertical direction and an extension direction of the end section 221b-2, the second protrusion 231b jacks up the extension section 221b of the conductive sheet 221. Specifically, the extension section 221b of the conductive sheet 221 deflects relative to the fixed terminal 221a until a highest point of the extension section 221b of the conductive sheet 221 in the vertical direction presses against the circuit board 22. This pressing point is a position at the conductive contact surface 222 is provided. In other words, the linear motion of the sliding member 23 towards the rear housing 25 is converted into a deflection motion of the conductive sheet 221. As mentioned above, since the fixed terminal 221a and the extension section 221b are conductive to each other, the discharging circuit is thereby turned on to form a closed loop.

Referring to FIG. 6c and FIG. 6d, when the sliding member 23 is at the second position, the second protrusion 231b of the sliding member 23 linearly moves towards the rear housing 25 and crosses the tip end 221b-3 of end section, so that the tip end 221b-3 of end section is put on the second working surface 233. Thus, the sliding member 23 causes the conductive sheet 221 to remain in a deflected state, that is, the highest point of the conductive sheet 221 continuously presses against the conductive contact surface 222. The advantage of this structure is that the second protrusion 231b crosses the tip end 221b-3 of end section to prevent the sliding member 23 from moving towards the front housing 21 and thus prevent the highest point of the conductive sheet 221 from being disconnected from the conductive contact surface 222.

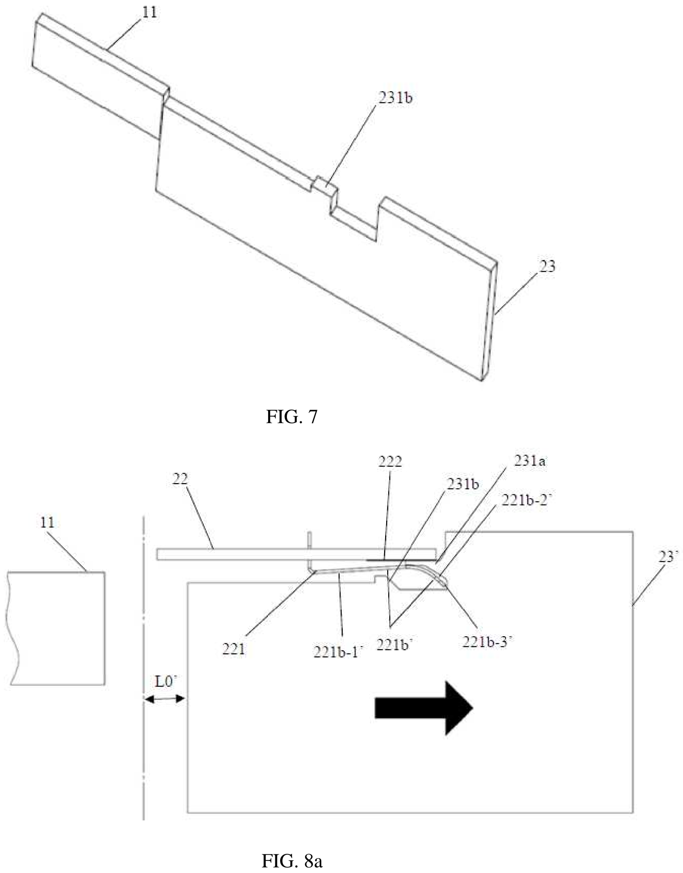

Referring to FIG. 7, the power source assembly base is provided with the abutting top portion 11. Specifically, the abutting top portion 11 extends out from the power source assembly base towards the power source assembly 2 in the slidable assembling direction, so as to press against the sliding member 23 in the assembling process. In this embodiment, the sliding member 23 is designed in a plate shape, so as to be placed in a gap between the two columns of power sources 24. To ensure that the abutting top portion 11 can also linearly move relative to the power source assembly 2 in this gap, the abutting top portion 11, especially its cross-sectional shape, is set to be similar to the plate shape of the sliding member 23, so that the abutting top portion 11 can enter the power source assembly 2, between the two columns of power sources 24, and cooperate with the sliding member 23 during the assembling.

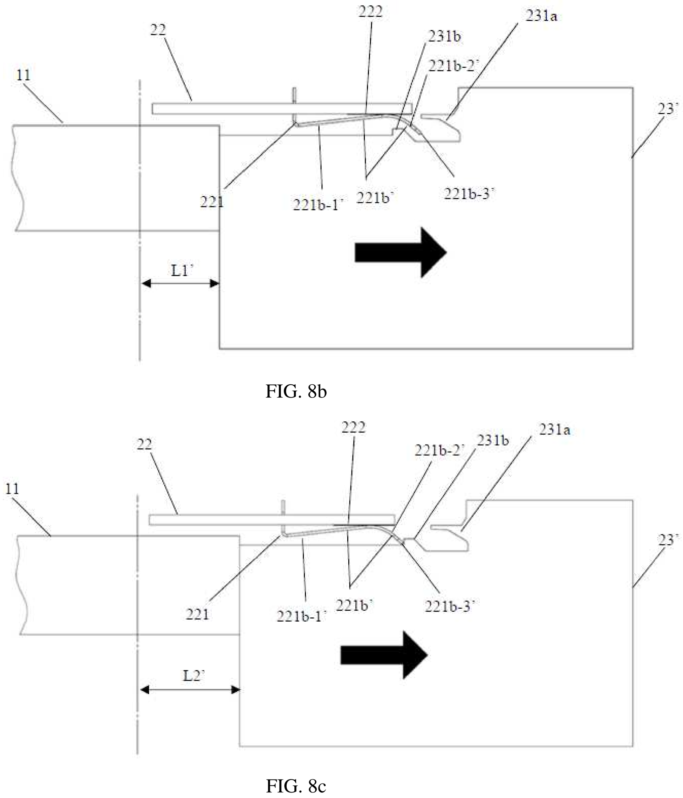

Referring to FIG. 8a, FIG. 8b, and FIG. 8c, another embodiment of the present application is shown, which differs from the previous embodiments in that the transition section 221b-1' does not extend in a direction parallel to the linear motion direction of the sliding member, but gradually approaches the circuit board 22 in the vertical direction while extending towards the rear housing 25. The end section 221b-2' still has the inclined downward extension presented, namely, it gradually approaches the sliding member 23 in the vertical direction while extending towards the rear housing 25. In this regard, the conductive sheet 221, especially its extension section 221b', and the conductive contact surface 222 form a normally closed switch, which means that in the absence of any interfering object, the extension section 221b' remains in contact with the conductive contact surface 222, thereby ensuring that the discharging circuit is in the turned-on state. To this end, the sliding member 23' is designed to have a first protrusion 231a, The first protrusion 231a extends between the extension section 221b' and the conductive contact surface 222, so that when the sliding member 23' is at the first position, that is, when the sliding member 23' is at its starting position, the first protrusion 231a is between the extension section 221b' and the conductive contact surface 222, to ensure the disconnection between the extension section 221b' and the conductive contact surface 222 and thus ensure that the discharging circuit is in the turned-off state, as shown in FIG. 8a.

Referring to FIG. 8b, in an assembling process that the power source assembly 2 slides into the power source assembly base along the above guide rail, the power source assembly 2 is at the middle position. The abutting top portion 11 presses against the sliding member 23', to cause the sliding member 23' to remain stationary relative to the surgical instrument 1. Relatively speaking, the first protrusion 231a is no longer between the extension section 221b' and the conductive contact surface 222. Based on the structure of the normally closed switch, the extension section 221b' and the conductive contact surface 222 are in contact with each other, thereby causing the discharging circuit to be in the turned-on state.

Referring to FIG. 8c, when the sliding member 23' is at the second position, the second protrusion 231b of the sliding member 23' linearly moves towards the rear housing 25 and crosses the tip end 221b-3' of end section. Thus, the second protrusion 231b prevents the sliding member 23' from moving towards the front housing 21.

It should be understood that the above accompanying drawings and specific implementations only describe exemplary embodiments of the present application, and are not exhaustive of possible implementations of the present application. Those skilled in the art can make various modifications to the above specific implementations within the scope of protection of the present application without departing from the spirit of the present application.

## Claims

1. A surgical instrument, comprising a power source assembly, a power source assembly base for accommodating the power source assembly, and a power source discharging mechanism, wherein the power source discharging mechanism is mounted on the power source assembly; **characterized in that**, the power source discharging mechanism comprises:
a circuit board, which comprises a discharging circuit, a conductive sheet, and a conductive contact surface that are integrated onto the circuit board, wherein the conductive sheet comprises a fixed terminal and an extension section; the fixed terminal of the conductive sheet is electrically connected to a first terminal of the discharging circuit; the extension section of the conductive sheet and the conductive contact surface switch between contact conduction and disconnection; the conductive contact surface is electrically connected to a second terminal of the discharging circuit; and
a sliding member, which is able to slide between a first position and a second position, wherein when the sliding member is at the first position, the extension section of the conductive sheet is disconnected from the conductive contact surface, and the discharging circuit is in a turned-off state; and when the sliding member is at the second position, the extension section of the conductive sheet is in contact conduction with the conductive contact surface, and the discharging circuit is in a turned-on state.

2. The surgical instrument according to claim 1, **characterized in that**, the fixed terminal is constructed as being rigid, and at least a portion of the extension section is constructed as being flexible, to cause the extension section to deflect relative to the fixed terminal and make the conductive sheet to be in contact with the conductive contact surface.

3. The surgical instrument according to claim 2, **characterized in that**, the extension section comprises a transition section and an end section; the transition section extends in a direction parallel to a linear motion direction of the sliding member, and connects the fixed terminal to the end section; and the transition section is farther away from the sliding member in a vertical direction than the end section.

4. The surgical instrument according to claim 3, **characterized in that**, the sliding member has a first working surface and a second working surface; when the sliding member is at the first position, a tip end of the end section is located on the first working surface; when the sliding member is at the second position, the tip end of the end section is located on the second working surface; and a height of the first working surface is lower than a height of the second working surface in the vertical direction.

5. The surgical instrument according to claim 2, **characterized in that**, the extension section is designed to cause the extension section and the conductive contact surface to be in a form of a normally closed switch.

6. The surgical instrument according to claim 5, **characterized in that**, the sliding member comprises a first protrusion designed to be located between the extension section and the conductive contact surface when the sliding member is at the first position, to ensure disconnection between the extension section and the conductive contact surface.

7. The surgical instrument according to claim 4 or 6, **characterized in that**, a second protrusion facing the conductive sheet is arranged on the second working surface of the sliding member; and when the sliding member is at the second position, the second protrusion prevents the sliding member from sliding towards the first position.

8. The surgical instrument according to claim 2, **characterized in that**, the conductive contact surface is positioned at a location on the circuit board where, when the sliding member is at the second position, at least a portion of the extension section of the conductive sheet presses against the circuit board.

9. The surgical instrument according to claim 1, **characterized in that**, the circuit board further comprises an input terminal and an output terminal that are respectively configured to be electrically connected to a positive electrode of a power source and a negative electrode of the power source; the input terminal is electrically connected to a third terminal of the discharging circuit; the output terminal is electrically connected to a fourth terminal of the discharging circuit; and the discharging circuit comprises a discharging resistor.

10. The surgical instrument according to claim 9, **characterized in that**, the power source assembly base comprises a first electrical contact terminal and a second electrical contact terminal; the first electrical contact terminal is configured to be electrically connected to the input terminal of the circuit board; and the second electrical contact terminal is configured to be electrically connected to the output terminal of the circuit board.

11. The surgical instrument according to claim 9 or 10, **characterized in that**, the power source assembly base further comprises a abutting top portion for pressing against the sliding member, so that when the power source assembly is assembled to the surgical instrument, the sliding member slides relative to the power source assembly, and the abutting top portion has the same shape as or a shape similar to a cross-sectional shape of the sliding member.

12. An assembling method for the surgical instrument according to any one of claims 1 to 4 and 7 to 11, wherein the power source assembly base has a guide rail, and the assembling method comprises:
- placing the power source assembly at a first position of the power source assembly base, wherein a abutting top portion is separated from the sliding member, the conductive sheet is put on the sliding member, and the extension section of the conductive sheet is separated from the conductive contact surface, to cause the discharging circuit to be in a turned-off state;
- pushing the power source assembly forwards along the guide rail to reach a middle position, wherein the abutting top portion presses against the sliding member and remains relatively stationary with the sliding member; the second protrusion of the sliding member presses against an end section of the extension section and forces the conductive sheet to elastically deform, and the extension section deflects relative to the fixed terminal until the extension section presses against the conductive contact surface, to cause the discharging circuit to be in a turned-on state; and
- continuing to push the power source assembly forwards along the guide rail to reach a second position, wherein the second protrusion moves and crosses the tip end of the end section to prevent the sliding member from moving towards the first position and then prevent the conductive sheet from being disconnected from the conductive contact surface, to cause the discharging circuit to remain in the turned-on state.
